(19) Europäisches Patentamt
European Patent Office
Office européen des brevets

(11) EP 1 535 604 B1

(12) # EUROPÄISCHE PATENTSCHRIFT

(45) Veröffentlichungstag und Bekanntmachung des Hinweises auf die Patenterteilung:
**01.07.2015 Patentblatt 2015/27**

(51) Int Cl.:
***A61K 8/39*** *(2006.01)* ***A61K 8/44*** *(2006.01)*
***A61Q 19/00*** *(2006.01)* ***A61Q 19/08*** *(2006.01)*
***A61K 31/198*** *(2006.01)* ***A61Q 17/00*** *(2006.01)*

(21) Anmeldenummer: **04026788.2**

(22) Anmeldetag: **11.11.2004**

(54) **Wirkstoffkombination aus Kreatin und Kreatinin, Phenoxyethanol und Glycerin**

Combination of creatine and creatinine, phenoxyethanol and glycerine

Combinaison à base de créatine et de créatinine, de phenoxyethanol et de glycérine.

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **26.11.2003 DE 10355714**

(43) Veröffentlichungstag der Anmeldung:
**01.06.2005 Patentblatt 2005/22**

(73) Patentinhaber: **Beiersdorf AG**
**20253 Hamburg (DE)**

(72) Erfinder:
- **Raschke, Thomas, Dr.**
  **25421 Pinneberg (DE)**
- **Mummert, Christopher, Dr.**
  **29553 Bienenbüttel (DE)**
- **Kallmayer, Volker, Dr.**
  **22525 Hamburg (DE)**

(56) Entgegenhaltungen:
**WO-A-00/15187**
**WO-A-03/005988**
**WO-A-03/011241**
**WO-A-03/020235**
**US-A- 5 422 112**

- **PATENT ABSTRACTS OF JAPAN & JP 2000 247866 A (LION CORPORATION), 12. September 2000 (2000-09-12)**
- **DATABASE GNPD [Online] MINTEL 10114241, Juli 2002 'Super Line Preventor +' Database accession no. 10114241**
- **DATABASE GNPD [Online] MINTEL 198043, März 2003 'Protek Performance Women's Shampoo Range' Database accession no. 198043**
- **DATABASE GNPD [Online] MINTEL 10149404, September 2003 'Straight & Sleek Conditioner' Database accession no. 10149404**
- **DATABASE GNPD [Online] MINTEL 228017, September 2003 'Retarding Body Hair Cream' Database accession no. 228017**
- **DATABASE GNPD [Online] MINTEL 10100141, Januar 2002 'Vitality Oxygen Complex Moisturizer' Database accession no. 10100141**

Bemerkungen:
Die Akte enthält technische Angaben, die nach dem Eingang der Anmeldung eingereicht wurden und die nicht in dieser Patentschrift enthalten sind.

EP 1 535 604 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft eine Wirkstoffkombination aus Kreatin Kreatinin Phenoxyethanol Glycerin, welche vorteilhaft in kosmetischen oder dermatologischen Zubereitungen zur Behandlung und Prophylaxe der Symptome von UV- und/oder Ozon- induzierten Hautschäden sowie von entzündlichen und degenerativen Hautzuständen verwendet werden kann.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind.einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Die vorteilhafte prophylaktische und therapeutische Wirkung des Kreatins bei der kosmetischen und medizinischen Hautpflege ist an sich bekannt. Kreatin (von grch.: το κρεα = "das Fleisch") zeichnet sich durch folgende Struktur aus:

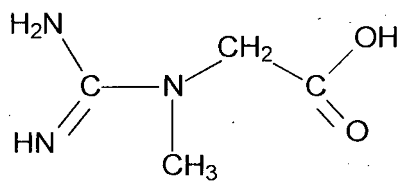

Es findet sich im Muskelsaft der Wirbeltiere zu 0,05-0,4%, in geringen Mengen auch im Gehirn und Blut. Als Monohydrat stellt es ein farbloses, kristallines Pulver dar. In wässriger Lösung wird Kreatinin gebildet.. Im Organismus entsteht es durch Transamidinierung von L-Arginin auf Glycin zu Guanidinoessigsäure und deren anschließende Methylierung mittels S-Adenosylmethionin (durch Guanidinoacetat-Methyltransferase). Man hält Kreatin für einen appetitfördernden Bestandteil von Rindfleisch und Fleischextrakt. Kreatinzusatz zur Nahrung verstärkt die körperliche Leistungsfähigkeit.

Umfangreich ist der Stand der Technik zu den kosmetischen und dermatologischen Verwendungen des Kreatins.

So beschreibt die DE 100 32 964 die Verwendung von Kreatin und/oder Kreatinderivaten in kosmetischen oder dermatologischen Zubereitungen zur Behandlung und Prophylaxe der Symptome von UV- und oder Ozon- induzierten Hautschäden sowie von entzündlichen und degenerativen Hautzuständen.

Die JP2000/247866 beschreibt Hautkosmetika mit einem Gehalt an Kreatin und/oder Kreatinin, welche als Creme oder als milchige Lotion verwendet werden können, wobei den betreffenden Zubereitungen vorzügliche hautpflegende Eigenschaften zugeschrieben werden. Ferner beschreibt die WO00/33787 die Verwendung von Kreatinin als wirksamen Bestandteil von Desodorantien.

Weiterhin beschreibt die EP-A 565 010 Haarwuchs- und Haarfärbezubereitungen mit einem Gehalt an Kreatininphosphat.

Schließlich werden in der US-A 4,590,067 und der EP-A-178 602 die Verwendung von Kreatin bzw. Kreatinin zur Herstellung von Zubereitungen mit antiinflammatorischer Wirksamkeit beschrieben.

Nachteilig ist jedoch, dass Kreatin und Kreatinin in wasserhaltigen Produkten leicht auskristallisieren, wobei Kristalle mit unkosmetischer Anmutung entstehen und die Wirksamkeit des Produktes vermindert wird. Diese Kristallisationsneigung wird verstärkt durch wasserlösliche Stoffe, deren Solubilisierung Wasser bindet, das somit nicht mehr zur Solubilisierung von Kreatin, Kreatinin und/oder deren Derivaten zur Verfügung steht.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Eine weitere Aufgabe bestand darin, eine Darreichungsform für Kreatin zu finden, die sich durch verminderte Neigung zur Bildung von Kreatinkristallen auszeichnet.

Es hat sich überraschenderweise herausgestellt, dass eine Wirkstoffkombination aus

(a) Kreatin und Kreatinin
(b) Phenoxyethanol
(c) Glycerin

wobei das Gewichtsverhältnis von Kreatinin zu Kreatin aus dem Bereich von 10 : 1 bis 1 : 10, besonders bevorzugt von 4 : 1 bis 3 zu 7, ganz besonders bevorzugt 2 : 1 bis 1 : 2 gewählt wird , sowie eine kosmetische oder dermatologische Zubereitung mit einem wirksamen Gehalt an einer solchen Wirkstoffkombination diese Aufgaben lösen.

Die WO 03/020235 A konnte nicht den Weg zur Erfindung weisen.

Werden Derivate des Kreatins eingesetzt, so ist das bevorzugte Derivat das Kreatinphosphat, welches folgende Struktur aufweist:

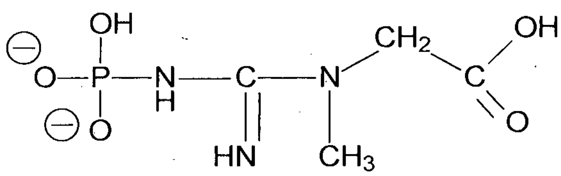

und welches im frischen Muskel verbreitet ist und dort als energiespeicherndes Phosphat (Phosphagen) eine wichtige Rolle spielt. Im arbeitenden Muskel gibt Kreatinphosphat mit Adenosin-5'-diphosphat unter dem Einfluss des Enzyms Kreatin-Kinase Adenosin-5' triphosphat (ATP) und Kreatin; im ruhenden Muskel läuft die umgekehrte Reaktion ab.

Aber auch Kreatinsulfat, Kreatinacetat, Kreatinascorbat und die an der Carboxylgruppe mit mono- oder polyfunktionalen Alkoholen veresterten Derivate führen zu vorteilhaften Ausführungsformen der Erfindung.

Kreatinin (ebenfalls von grch.: το κρεα = "das Fleisch") ist durch folgende Struktur gekennzeichnet

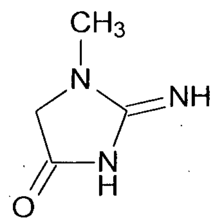

und entsteht im Organismus durch nichtenzymatische Umwandlung aus Kreatinphosphat gemäß

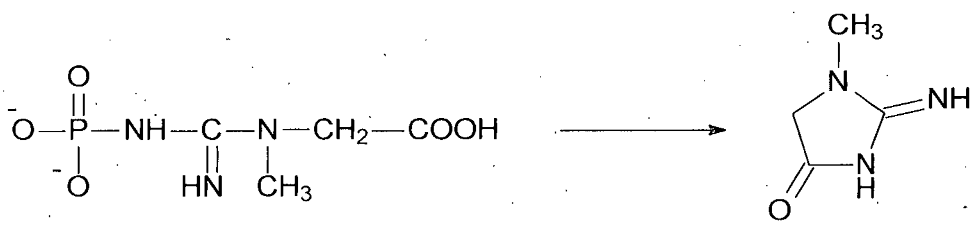

und wird über die Niere ausgeschieden. Die Menge der Kreatininausscheidung ist der Muskelmasse proportional und für das jeweilige Individuum annähernd konstant. Kreatinin ist in Fleischextrakt und Fleischbrühwürfeln enthalten.

Bevorzugt enthalten kosmetische oder dermatologische Zubereitungen gemäß der Erfindung 0,0001 - 10 Gew.-%, besonders bevorzugt 0,001 - 1 Gew.-%, an Kreatinin und/oder Kreatininderivaten, bezogen auf die Gesamtzusammensetzung der Zubereitungen.

Phenoxyethanol ist durch die chemische Struktur

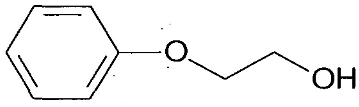

gekennzeichnet und stellt eine viskose Flüssigkeit von schwachem, leicht angenehmen Geruch und einem zusammenziehenden Geschmack dar. Phenoxyethanol wurde in der Natur nachgewiesen in tropischen Früchten, in Cichorium endivia sowie in grünem Tee (Camellia sinesis). Es hat einen milden, rosenähnlichen Duft und wird für Parfümkompositionen auch als Fixatur eingesetzt. Es ist mischbar mit Aceton, Ethylalkohol und Glycerin, löslich in Wasser und Fetten, z.B. Oliven- und Erdnussöl.

Phenoxyethanol ist vor allem in saurem und neutralem, aber auch im alkalischen Milieu wirksam und völlig ungiftig. Es gibt bereits in niedrigen Konzentrationen ausreichend Schutz. Aufgrund seiner guten Verträglichkeit verbunden mit seiner hervorragenden Wirksamkeit fand es schnell Eingang in die pharmazeutische und kosmetische Industrie.

Die Verwendung von Glycerin in Kosmetika ist allgemein bekannt. Glycerin wirkt hautbefeuchtend und hautglättend und ist Bestandteil vieler hautpflegender kosmetischer Zubereitungen.

Vorteilhaft enthält eine erfindungsgemäße Zubereitung, enthaltend 0,001 - 30 Gew.-%, besonders bevorzugt 0,01 - 15 Gew.-%, ganz besonders bevorzugt 1 - 7 Gew.-% Glycerin, bezogen auf die Gesamtzusammensetzung der Zubereitung.

Ein großes Problem ist aber, daß Glycerin und Phenoxyethanol die Kristallisationsneigung von wasserlöslichen Wirkstoffen in kosmetischen Zubereitungen wie Hydrogelen, W/O-Emulsionen oder O/W-Emulsionen verstärken, was zu unkosmetischer Anmutung und/oder Wirkverlust während der Lagerung oder des Gebrauchs führen kann.

Aufgabe der vorliegenden Erfindung war es somit, Wege zu finden, die die Nachteile des Standes der Technik vermeiden. Insbesondere sollen Zubereitungen zur Behebung der mit der endogenen, chronologischen und exogenen Hautalterung verbundenen Schäden und zur dauerhaften Prophylaxe bereitgestellt werden, die auch in Hinblick auf mögliche Verkeimung des Produktes sicher sind.

Eine weitere Aufgabe bestand darin, eine Darreichungsform für Kreatin zu finden, die sich durch verminderte Neigung zur Bildung von Kreatinkristallen auszeichnet.

Es hat sich überraschenderweise herausgestellt, dass erfindungsgemäße Wirkstoffkombinationen sowie eine kosmetische oder dermatologische Zubereitung mit einem wirksamen Gehalt an solchen Wirkstoffkombinationen diese Aufgaben lösen.

Als ganz besonders vorteilhaft hat sich erwiesen, wenn die erfindungsgemäße Zubereitung dadurch gekennzeichnet ist, dass die nachfolgenden Gewichtsverhältnisse eingestellt werden:

> (A : B : C) wie a : b : c gewählt wird, wobei a, b und c unabhängig voneinander positive rationale Zahlen von 1 bis 200, bevorzugt von 1 bis 50 darstellen.
> A stellt die Summe der Konzentrationen von Kreatin und Kreatinin in Gewichtseinheiten (z.B. Gew.-%) dar,
> B stellt die Konzentration von Phenoxyethanol in denselben Gewichtseinheiten dar,
> C stellt die Konzentration von Glycerin in denselben Gewichtseinheiten dar, jeweils bezogen auf das Gesamtgewicht der Zubereitung.

Ferner hat es sich als vorteilhaft erwiesen, den Quotienten

$$(B + C) / A$$

wobei A, B, und C die zuvor beschriebenen Eigenschaften aufweisen,
aus dem Bereich zwischen 0,5 und 200, bevorzugt aus dem Bereich zwischen 1 und 50 zu wählen.

Erfindungsgemäß werden die Wirkstoffkombinationen bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind.

Die erfindungsgemäß verwendeten Wirkstoffkombinationen lassen sich ohne Schwierigkeiten in gängige kosmetische oder dermatologische Formulierungen einarbeiten, vorteilhaft in Emulsionen, Pumpsprays, Aerosolsprays, Aerosolemulsionsschäumen, Cremes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, die erfindungsgemäß verwendeten Wirkstoffkombinationen mit anderen Wirkstoffen zu kombinieren, beispielsweise mit anderen antimikrobiell, antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 8,0. Besonders günstig ist es, den pH-Wert in einem Bereich von 6,5 - 8,0 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Entsprechend können kosmetische und/oder topische dermatologische Zusammensetzungen im Sinne der vorliegenden Erfindung, je nach ihrem Aufbau, beispielsweise verwendet werden als Hautpflegeprodukt, Hautschutzprodukt, Reinigungsprodukt, Sonnenschutzprodukt, Haarkur, Körperreinigungsprodukt, für die Tages- oder Nachtpflege, die Pflege bestimmter Hautareale wie Hände, Gesicht, Füße usw.

Auch ist die Verwendung der erfindungsgemäßen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut erfindungsgemäß.

Weiterhin ist die Verwendung der erfindungsgemäßen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rizinusöl), Ceramide, insbes. Ceramid I, III und VI, Cholesterin, Phytosterole, Fettsäuren mit einer Kettenlänge von $C_{16-26}$, Carnitin und seine Derivate, Harnstoff, Polyole wie Glycerin, Butylenglykol, Propylenglykol und Dipropylenglykol, Pseudoceramide; Elektrolyte wie Natriumchlorid und Taurin, Fettalkohole sowie Wachse.

Außerdem ist die Verwendung der erfindungsgemäßen Zubereitungen zur Prophylaxe und Behandlung der Symptome der fehlpigmentierten Haut vorteilhaft. Bevorzugte Wirkstoffe für diesen Einsatzzweck sind: Tyrosinaseinhibitoren, Azelainsäure, Hydrochinonderivate, Dioic Acid, Liponsäure und ihre Derivate sowie Kojisäure.

Nicht zuletzt ist die Verwendung der erfindungsgemäßen kosmetischen und/oder dermatologischen Zubereitungen zur Prophylaxe, Behandlung und Reinigung fettiger Haut sowie zur Prophylaxe und Behandlung von unreiner Haut sowie von Cellulite erfindungsgemäß.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Ölin-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:

- Wasser oder wässrige Lösungen;
- Öle, wie Triglyceride der Caprin- oder der Caprylsäure, Fettsäureether wie Dicaprylylether, Kohlensäureester wie Dicaprylylcarbonat, sowie pflanzliche Triglyceride wie Sonnenblumenöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether, Ethylhexylglycerin, Methylpropandiol und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxy-zimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie

die Sulfonsäure selbst;

- Sulfonsäure-Derivate von Benzophenonen, vorzugsweise 2-Hydroxy-4-methoxyben-zophenon-5-sulfonsäure und ihre Salze;
- Sulfonsäure-Derivate des 3-Benzylidencamphers, wie z.B. 4-(2-Oxo-3-bornyliden-methyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornyli-denmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Emulsionen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion.

Weiterhin vorteilhafte UVA-Filter entstammen der Gruppe der Triazine, so z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handels-bezeichnung Tinosorb® M). Ein vorteilhafter wasserlöslicher UVA-Filter stellt das 2'-bis-(1,4-Phenylen)-1 H-benzimidazol-4,6-disulfonsäure-Natriumsalz dar (Handelsbezeichnung Neo Heliopan AP®). Es können die für die UVB-Kombination verwendeten Mengen eingesetzt werden.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im Allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$RNH_2^+CH_2CH_2COOH\ X^-$ (bei pH=2) $X^-$ = beliebiges Anion, z.B. $Cl^-$

$RNH_2^+CH_2CH_2COO^-$ (bei pH=7)

$RNHCH_2CH_2COO^-\ B^+$(bei pH=12) $B^+$ = beliebiges Kation, z.B. $Na^+$

Typisch für nicht-ionische Tenside sind Polyether-Ketten. Nicht-ionische Tenside bilden in wässrigem Medium keine Ionen.

A. Anionische Tenside

Vorteilhaft zu verwendende anionische Tenside sind
Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate

Carbonsäuren und Derivate, wie

1. Carbonsäuren, beispielsweise Laurinsäure, Aluminiumstearat, Magnesiumalkanolat und Zinkundecylenat,

2. Ester-Carbonsäuren, beispielsweise Calciumstearoyllactylat, Laureth-6 Citrat und Natrium PEG-4 Lauramidcarboxylat,

3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie
Schwefelsäureester, wie

1. Alkylethersulfat, beispielsweise Natrium-, Ammonium-, Magnesium-, MIPA-, TIPA- Laurethsulfat, Natriummyrethsulfat und Natrium $C_{12-13}$ Parethsulfat,
2. Alkylsulfate, beispielsweise Natrium-, Ammonium- und TEA- Laurylsulfat.

B. Kationische Tenside

Vorteilhaft zu verwendende kationische Tenside sind

1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.

Die Lipidphase der erfindungsgemäßen kosmetischen oder dermatologischen Emulsionen kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße, als Emulsionen vorliegende Zubereitungen enthalten einen oder mehrere Emulgatoren. O/W-Eulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R-O-(-CH_2-CH_2-O-)_n-R'$,
- der Fettsäureethoxylate der allgemeinen Formel
- $R-COO-(-CH_2-CH_2-O-)_n-H$,
- der veretherten Fettsäureethoxylate der allgemeinen Formel
- $R-COO-(-CH_2-CH_2-O-)_n-R'$,
- der veresterten Fettsäureethoxylate der allgemeinen Formel
- $R-COO-(-CH_2-CH_2-O-)_n-C(O)-R'$,
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel $R-O-(-CH_2-CH_2-O-)_n-CH_2-COOH$ nd n eine Zahl von 5 bis 30 darstellen,
- der Polyoxyethylensorbitolfettsäureester,
- der Alkylethersulfate der allgemeinen Formel $R-O-(-CH_2-CH_2-O-)_n-SO_3-H$
- der Fettalkoholpropoxylate der allgemeinen Formel
- $R-O-(-CH_2-CH(CH_3)-O-)_n-H$,
- der Polypropylenglycolether der allgemeinen Formel
- $R-O-(-CH_2-CH(CH_3)-O-)_n-R'$.
- der propoxylierten Wollwachsalkohole,
- der veretherten Fettsäurepropoxylate
- $R-COO-(-CH_2-CH(CH_3)-O-)_n-R'$,
- der veresterten Fettsäurepropoxylate der allgemeinen Formel
- $R-COO-(-CH_2-CH(CH_3)-O-)n-C(O)-R'$,
- der Fettsäurepropoxylate der allgemeinen Formel
- $R-COO-(-CH_2-CH(CH_3)-O-)_n-H$,
- der Polypropylenglycolglycerinfettsäureester -
- der propoxylierten Sorbitanester
- der Cholesterinpropoxylate
- der propoxylierten Triglyceride
- der Alkylethercarbonsäuren der allgemeinen Formel
- $R-O-(-CH_2-CH(CH_3)O-)_n-CH_2-COOH$

- der Alkylethersulfate bzw. die diesen Sulfaten zugrundeliegenden Säuren der allgemeinen Formel R-O-(-$CH_2$-$CH(CH_3)$-O-)$_n$-$SO_3$-H
- der Fettalkoholethoxylate/propoxylate der allgemeinen Formel
- R-O-$X_n$-$Y_m$-H,
- der Polypropylenglycolether der allgemeinen Formel
- R-O-$X_n$-$Y_m$-R',
- der veretherten Fettsäurepropoxylate der allgemeinen Formel
- R-COO-Xn-Ym-R',
- der Fettsäureethoxylate/propoxylate der allgemeinen Formel
- R-COO-$X_n$-$Y_m$-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20) stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15) isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol-(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)-cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)iso-stearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol (18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäße Zubereitungen können vorteilhaft zusätzlich zu Phenoxyethanol weitere Konservierungsmittel enthalten. Dadurch können die Konzentrationen der einzelnen Konservierungsmittel reduziert werden, was oft einen vorteilhaften Einfluss auf die Hautverträglichkeit der Zubereitung hat, oder es können Konservierungsmittel unterschiedlicher Wirkungsprofile miteinander kombiniert und so der Gesamtschutz der Zubereitung entscheidend verbessert werden.

Vorteilhaft lassen sich zum verbesserten Schutz der erfindungsgemäßen Zubereitung in Kombination mit Phenoxyethanol einsetzen Sorbinsäure und ihre Salze, Ester der para-Hydroxybenzoesäure (Parabene), vorteilhaft die Methyl-, Ethyl-, Propyl-, Butyl- und Isobutylester der para-Hydroxybenzoesäure, bespnders vorteilhaft Mischungen dieser Ester, Dibromhexamidin2-Brom-2-nitro-1,3-propandiol, Imidazolidinylharnstoff, Polyaminopropylbiguanid, 1,3-Bis-(hydroxymethyl)-5,5-dimethyl-2,4-imidazolidindion (DMDM Hydantoin), Methylchloroisothiazolinon, Methylisothiazolinon, Diazolidinylharnstoff, Benzoesäure, Benzylalkohol, lodopropinylbutylcarbamat sowie Mischungen dieser Verbindungen.

Die folgenden Beispiele sollen die Erfindung erläutern, aber nicht einschränken. Die Zahlenangaben beziehen sich auf Gew.-%, sofern nichts anderes angegeben ist.

| **Beispiel 1 O/W-Emulsion** | **Gew.-%** |
|---|---|
| Glycerylstearat | 1 |
| Stearinsäure | 3 |
| Stearylalkohol | 3 |
| Cetylalkohol | 2 |
| $C_{12-15}$ Alkylbenzoat | 2 |

(fortgesetzt)

| **Beispiel 1 O/W-Emulsion** | **Gew.-%** |
|---|---|
| Caprylsäure/Caprinsäure Triglycerid | 2 |
| Macadamiaöl | 1 |
| Sheabutter | 2 |
| Bienenwachs | 1 |
| Dimethicone | 1 |
| Hydrierte Kokosfettglyceride (Hydrogenated Coco Glycerides) | 1 |
| Ethylhexylglycerin | 0,5 |
| Tocopherylacetat | 1 |
| Kreatinin | 0,1 |
| Kreatin | 0,8 |
| Ubichinon (Q10) | 0,03 |
| Retinylpalmitat | 0,1 |
| Phenoxyethanol | 0,2 |
| p-Hydroxybenzoesäurealkylester (Paraben) | 0,3 |
| Iodopropinylbutylcarbamat | 0,02 |
| Cyclodextrin | 0,5 |
| Iminodisuccinat | 0,2 |
| Carbomer | 0,3 |
| Glycerin | 5 |
| Butylenglycol | 1 |
| Methylpropandiol | 1 |
| Additive ($SiO_2$, BHT, Talkum) | 0,5 |
| Parfüm | q.s. |
| Wasser | ad 100 |

| **Beispiel 2 O/W-Emulsion** | **Gew.-%** |
|---|---|
| Glycerylstearat | 2 |
| PEG-40-Stearat | 1 |
| Myristylmyristat | 1 |
| Cetearylalkohol | 2 |
| Sheabutter | 2 |
| $C_{12-15}$ Alkylbenzoat | 3 |
| Caprylsäure/Caprinsäure Triglycerid | 2 |
| Ethylhexylkokosfettsäureester | 1 |
| Vaseline | 2 |
| Cyclomethicon | 5 |
| $TiO_2$ | 1 |

(fortgesetzt)

| **Beispiel 2 O/W-Emulsion** | **Gew.-%** |
|---|---|
| Ethylhexylmethoxycinnamat | 3 |
| 2-Hydroxy-4-Methoxy-benzophenon (Oxybenzone) | 2 |
| Ubichinon (Q10) | 0,05 |
| Tocopherylacetat | 0,5 |
| Kreatinin | 0,2 |
| Kreatin | 1,0 |
| Retinylpalmitat | 0,1 |
| Natrium-Ascorbylphosphat | 0,1 |
| Phenoxyethanol | 0,2 |
| p-Hydroxybenzoesäurealkylester (Paraben) | 0,3 |
| Polyacrylsäure (Carbomer) | 0,1 |
| Aluminium Stärke Octenylsuccinate | 0,5 |
| Glycerin | 5 |
| Füllstoffe/ Additive (Distärkephosphat, $SiO_2$, BHT, Talkum, Aluminiumstearat) | 0,05 |
| Parfüm | q.s. |
| Wasser | ad 100 |

| **Beispiel 3 O/W-Emulsion** | **Gew.-%** |
|---|---|
| Glycerylsterat | 2,5 |
| PEG-40-Stearat | 1 |
| Cetearylalkohol | 2 |
| Hydrierte Kokosfettglyceride (Hydrogenated Coco Glycerides) | 1 |
| Sheabutter | 2 |
| $C_{12-15}$ Alkylbenzoat | 4 |
| Caprylsäure/Caprinsäure Triglycerid | 2 |
| Octyldodecanol | 1 |
| Vaseline | 1 |
| Dicaprylylcarbonat | 3 |
| $TiO_2$ | 1 |
| Ethylhexylcyanodiphenyl-acrylat (Octocrylen) | 5 |
| Phenylbenzimidazol-sulfonsäure | 1 |
| 2-Hydroxy-4-Methoxy- benzophenon (Oxybenzone) | 2 |
| Ubichinon (Q10) | 0,03 |
| Kreatinin | 0,02 |
| Kreatin | 0,05 |
| Cyclodextrin | 0,2 |
| Iminodisuccinat | 0,2 |

(fortgesetzt)

| Beispiel 3 O/W-Emulsion | Gew.-% |
|---|---|
| Phenoxyethanol | 0,5 |
| p-Hydroxybenzoesäurealkylester (Paraben) | 0,4 |
| Iodopropinylbutylcarbamat | 0,05 |
| 2-Ethylhexylglycerinether (Octoxyglycerin) | 0,5 |
| Polyacrylsäure (Carbomer) | 0,2 |
| Nylonmikropartikel | 1 |
| Glycerin | 10 |
| Additive (Distärkephosphat, $SiO_2$, Talkum, BHT, Aluminiumstearat) | 0.03 |
| Parfüm | q.s. |
| Wasser | ad 100 |

| Beispiel 4 O/W-Emulsion | Gew.-% |
|---|---|
| Polyglyceryl-3-Methylglucosedistearat | 2 |
| Cetylalkohol | 1 |
| $C_{12-15}$ Alkylbenzoat | 2 |
| Butylenglycol Dicaprylat/Dicaprat | 2 |
| Caprylsäure/Caprinsäure Triglycerid | 2 |
| Hydriertes Polydecen | 1 |
| Dimethylpolysiloxan (Dimethicon) | 1 |
| Isodecylneopentanoate | 4 |
| Kreatinin | 0,1 |
| Kreatin | 1 |
| Natriumascorbylphosphat | 0,1 |
| EDTA | 0,2 |
| Phenoxyethanol | 0,4 |
| Iodopropinylbutylcarbamat | 0,05 |
| p-Hydroxybenzoesäurealkylester (Paraben) | 0,4 |
| Ethanol denaturiert | 2 |
| Carbomer | 0,,2 |
| Iminodisuccinat | |
| Glycerin | 5 |
| Additive (Distärkephosphat, Talkum, BHT) | 0,2 |
| Parfüm | q.s. |
| Wasser | ad 100 |

| Beispiel 5 O/W-Emulsion | Gew.-% |
|---|---|
| Polyethylenglycol(21)stearyl-ether (Steareth-21) | 2 |

(fortgesetzt)

| Beispiel 5 O/W-Emulsion | Gew.-% |
|---|---|
| Polyethylenglycol(2)stearyl-ether (Steareth-2) | 1 |
| Cetearylalkohol | 2 |
| Sheabutter | 1 |
| $C_{12-15}$ Alkylbenzoat | 2 |
| Octyldodecanol | 1 |
| Mineralöl | 3 |
| Octamethyltetrasiloxan (Cyclomethicon) | 4 |
| Dicaprylylether | 2 |
| $TiO_2$ | 1 |
| Ethylhexylmethoxycinnamat | 4 |
| Ethylhexyltriazon | 1 |
| Ubichinon (Q10) | 0,02 |
| Kreatinin | 0,12 |
| Kreatin | 1,0 |
| Biotin | 0,02 |
| Trinatrium EDTA | 0,2 |
| Phenoxyethanol | 0,5 |
| p-Hydroxybenzoesäurealkylester (Paraben) | 0,3 |
| Iodopropinylbutylcarbamat | 0,1 |
| Polyacrylsäure (Carbomer) | 0,2 |
| Ammoniumacryloyldimethyltaurate/Vinylpyrrolidoncopolymere | 0,3 |
| Glycerin | 6 |
| Additive (Distärkephosphat, $SiO_2$, Talkum, BHT, Alumini-umstearat) | 0,05 |
| Parfüm | q.s. |
| Wasser | ad 100 |

## Patentansprüche

**1.** Wirkstoffkombination aus

(a) Kreatin und Kreatinin
(b) Phenoxyethanol
(c) Glycerin.

wobei das Gewichtsverhältnis von Kreatinin zu Kreatin aus dem Bereich von 10 : 1 bis 1 : 10, besonders bevorzugt von 4 : 1 bis 3 zu 7, ganz besonders bevorzugt 2 : 1 bis 1 : 2 gewählt wird.

**2.** Kosmetische oder dermatologische Zubereitung mit einem wirksamen Gehalt an einer Wirkstoffkombination gemäß Anspruch 1..

**3.** Zubereitung nach Anspruch 2, enthaltend 0,001 - 10 Gew.-%, besonders bevorzugt 0,01 - 1 Gew.-%, an Kreatin und/oder Kreatinderivaten, bezogen auf die Gesamtzusammensetzung der Zubereitung.

4. Zubereitung nach einem der vorstehenden Ansprüche, enthaltend 0,001 - 10 Gew.-%, besonders bevorzugt 0,01 - 1 Gew.-%, an Kreatinin und/oder Kreatininderivaten, bezogen auf die Gesamtzusammensetzung der Zubereitung.

5. Zubereitung nach einem der vorstehenden Ansprüche, enthaltend 0,001 - 30 Gew.-%, besonders bevorzugt 0,01 - 15 Gew.-%, an Glycerin, ganz besonders bevorzugt 1 - 7 Gew.-% Glycerin, bezogen auf die Gesamtzusammensetzung der Zubereitung.

6. Zubereitung nach einem der vorstehenden Ansprüche, **dadurch gekennzeichnet, dass** die nachfolgenden Gewichtsverhältnisse eingestellt werden:

   (A : B : C) wie a : b : c gewählt wird, wobei a, b und c unabhängig voneinander rationale Zahlen von 1 bis 200, bevorzugt von 1 bis 50 darstellen, wobei

   A die Summe der Konzentrationen von Kreatin und Kreatinin in Gewichtseinheiten (z.B. Gew.-%) darstellt,
   B die Konzentration von Phenoxyethanol in denselben Gewichtseinheiten darstellt,
   C die Konzentration von Glycerin in denselben Gewichtseinheiten darstellt,

   jeweils bezogen auf das Gesamtgewicht der Zubereitung.

7. Zubereitung nach einem der vorstehenden Ansprüche, **dadurch gekennzeichnet, dass** der Quotient

$$(B + C) / A$$

wobei

A die Summe der Konzentrationen von Kreatin und Kreatinin in Gewichtseinheiten (z.B. Gew.-%) darstellt,
B die Konzentration von Phenoxyethanol in denselben Gewichtseinheiten darstellt,
C die Konzentration von Glycerin in denselben Gewichtseinheiten darstellt,

jeweils bezogen auf das Gesamtgewicht der Zubereitung
aus dem Bereich zwischen 0,5 und 200, bevorzugt aus dem Bereich zwischen 1 und 50 gewählt wird.

## Claims

1. Active ingredient combination of

   (a) creatine and creatinine
   (b) phenoxyethanol
   (c) glycerol.

   wherein the weight ratio of creatinine to creatine is selected in the range of 10 : 1 to 1 : 10, particularly preferably 4 : 1 to 3 to 7, especially preferably 2 : 1 to 1 : 2.

2. Cosmetic or dermatological formulation having an effective content of an active ingredient combination according to Claim 1.

3. Formulation according to Claim 2, comprising 0.001 - 10% by weight, particularly preferably 0.01 - 1% by weight, of creatine and/or creatine derivatives, based on the total composition of the formulation.

4. Formulation according to any one of the preceding claims, comprising 0.001 - 10% by weight, particularly preferably 0.01 - 1% by weight, of creatinine and/or creatinine derivatives, based on the total composition of the formulation.

5. Formulation according to any one of the preceding claims, comprising 0.001 - 30% by , weight, particularly preferably 0.01 - 15% by weight, of glycerol, especially preferably 1 - 7% by weight of glycerol, based on the total composition of the formulation.

**6.** Formulation according to any one of the preceding claims, **characterized in that** the weight ratios are adjusted as follows:

(A : B : C) where a : b : c is selected, in which a, b and c are mutually independently rational numbers from 1 to 200, preferably from 1 to 50, wherein

A is the sum total of the concentrations of creatine and creatinine in units of weight (e.g. % by weight),
B is the concentration of phenoxyethanol in the same units of weight,
C is the concentration of glycerol in the same units of weight,
based in each case on the total weight of the formulation.

**7.** Formulation according to any one of the preceding claims, **characterized in that** the quotient

$$(B + C) / A$$

wherein

A is the sum total of the concentrations of creatine and creatinine in units of weight (e.g. % by weight),
B is the concentration of phenoxyethanol in the same units of weight,
C is the concentration of glycerol in the same units of weight,
based in each case on the total weight of the formulation

is selected in the range between 0.5 and 200, preferably in the range between 1 and 50.

**Revendications**

**1.** Combinaison de substances actives constituée par

(a) de la créatine et de la créatinine
(b) du phénoxyéthanol
(c) du glycérol,

le rapport pondéral de créatinine à créatine étant choisi dans la plage de 10 : 1 à 1 : 10, de manière particulièrement préférée de 4 : 1 à 3 : 7, de manière tout particulièrement préférée de 2 : 1 à 1 : 2.

**2.** Préparation cosmétique ou dermatologique présentant une teneur active en une combinaison de substances actives selon la revendication 1.

**3.** Préparation selon la revendication 2, contenant 0,001 - 10% en poids, de manière particulièrement préférée 0,01 - 1% en poids, de créatine et/ou de dérivés de créatine, par rapport à la composition totale de la préparation.

**4.** Préparation selon l'une quelconque des revendications précédentes, contenant 0,001 - 10% en poids, de manière particulièrement préférée 0,01 - 1% en poids, de créatinine et/ou de dérivés de créatinine, par rapport à la composition totale de la préparation.

**5.** Préparation selon l'une quelconque des revendications précédentes, contenant 0,001 - 30% en poids, de manière particulièrement préférée 0,01 - 15% en poids, de glycérol, de manière tout particulièrement préférée 1 - 7% en poids de glycérol, par rapport à la composition totale de la préparation.

**6.** Préparation selon l'une quelconque des revendications précédentes, **caractérisée par** le réglage des rapports pondéraux suivants :

(A:B:C) est choisi comme a:b:c, où a, b et c représentent, indépendamment l'un de l'autre, des nombres rationnels de 1 à 200, de préférence de 1 à 50,

A représentant la somme des concentrations de créatine et de créatinine en unités pondérales (par exemple en % en poids),
B représentant la concentration en phénoxyéthanol dans les mêmes unités pondérales,
C représentant la concentration en glycérol dans les mêmes unités pondérales,

à chaque fois par rapport au poids total de la composition.

7. Préparation selon l'une quelconque des revendications précédentes, **caractérisée en ce que** le quotient

$$(B + C) / A$$

A représentant la somme des concentrations de créatine et de créatinine en unités pondérales (par exemple en % en poids),
B représentant la concentration en phénoxyéthanol dans les mêmes unités pondérales,
C représentant la concentration en glycérol dans les mêmes unités pondérales,

à chaque fois par rapport au poids total de la composition,
est choisi dans la plage entre 0,5 et 200, de préférence dans la plage entre 1 et 50.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 10032964
- JP 2000247866 A
- WO 0033787 A
- EP 565010 A
- US 4590067 A
- EP 178602 A
- WO 03020235 A

### In der Beschreibung aufgeführte Nicht-Patentliteratur

- *Int. J. Cosm. Science,* 1988, vol. 10, 53
- **A. VOELCKEL et al.** *Zentralblatt Haut- und Geschlechtskrankheiten,* 1989, vol. 156, 2